(19) Europäisches Patentamt
European Patent Office
Office européen des brevets

(11) **EP 1 959 901 B1**

(12) # EUROPEAN PATENT SPECIFICATION

(45) Date of publication and mention of the grant of the patent:
**25.02.2015 Bulletin 2015/09**

(21) Application number: **05819070.3**

(22) Date of filing: **12.12.2005**

(51) Int Cl.:
***A61F 13/42*** *(2006.01)* ***G01N 27/00*** *(2006.01)*
***G08B 21/20*** *(2006.01)*

(86) International application number:
**PCT/SE2005/001907**

(87) International publication number:
**WO 2007/069945 (21.06.2007 Gazette 2007/25)**

(54) **ABSORBENT ARTICLE COMPRISING WETNESS DETECTING MEANS**

SAUGFÄHIGER ARTIKEL MIT EINEM MITTEL ZUM NACHWEIS VON NÄSSE

ARTICLE ABSORBANT COMPRENANT DES MOYENS DE DÉTECTION D'HUMIDITÉ

(84) Designated Contracting States:
**AT BE BG CH CY CZ DE DK EE ES FI FR GB GR HU IE IS IT LI LT LU LV MC NL PL PT RO SE SI SK TR**

(43) Date of publication of application:
**27.08.2008 Bulletin 2008/35**

(73) Proprietor: **SCA Hygiene Products AB**
**405 03 Göteborg (SE)**

(72) Inventors:
- **GUSTAFSON, Ingrid**
**S-430 31 Åsa (SE)**
- **BERLAND, Carolyn**
**S-431 36 Mölndal (SE)**

(74) Representative: **Valea AB**
**Box 1098**
**405 23 Göteborg (SE)**

(56) References cited:
**DE-A1- 3 437 950**
**US-A- 4 539 559**
**US-A- 5 903 222**
**US-A1- 2002 070 868**
**US-A1- 2003 060 789**
**US-A1- 2004 064 114**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 959 901 B1

## Description

TECHNICAL FIELD

The present invention concerns an absorbent article comprising wetness detecting means.

BACKGROUND OF THE INVENTION

Absorbent articles possessing different types of detecting means are known, and help to alert a user or caregiver to a change within the article (e.g. a soiling event). Such detecting means allow the user or caregiver to readily determine whether or not an absorbent article needs to be changed, without the need for close inspection or removal of the article.

Commonly known detecting means which can be incorporated into absorbent articles are chemical substances which alter their form or nature upon contact with liquid. For example, an indication that an absorbent article is soiled can arise from colour changes or the appearance or disappearance of an element on the absorbent article. Such technology is known from, e.g. US 5 389 093, WO 04/028403 and WO 05/030084. Such detecting means are useful in certain situations, but less so in institutions such as child-care centres, care centres for the elderly or hospitals where the status of a large number of wearers must be monitored, often by a limited staff. Determining whether the absorbent article is soiled or not still requires the wearer to be disturbed, as the coloured element must still be visible to the caregiver. This often requires that the wearer be moved, and their clothes removed or adjusted.

WO02/47592 describes an article having a status signalling device for communicating a change in status of a monitored portion. The signalling device can comprise a sensor located within the article, the sensor being connected to an external portion located on the outside of the article. Changes in the status of the article (e.g. soiling) can be transmitted from the signalling device to a receiver via an RF link produced by the external portion. The external portion is included on the outside of the article and is secured in place, e.g. by hook-and-loop type fasteners. As such, it can be removed or displaced and is subject to external influences (e.g. abrasion, moisture, interference by the wearer). Furthermore, traditional components of the external portion described in WO02/47592 render it comparatively expensive to produce, which in turn, renders its disposal expensive and reuse more likely.

US 2005/ 0 156 744 describes a diaper similar to that of WO02/47592, in which a detachable transmitter is installed on the outside of the diaper.

WO02/78513 describes a fluid discharge monitoring apparatus for a diaper. The apparatus comprises an RF tag which is responsive to the discharge of fluid into the diaper. There is no discussion in WO02/78513 as to the nature of the components which are used in the fabrication of the monitoring apparatus.

JP 2005000602 describes a wet detecting device in a diaper, comprising an RF-ID tag. The tag comprises an IC chip, a communication control section, data storage medium and an antenna.

WO 99/33037 discloses a method and apparatus for detecting a fluid, said method comprising providing one or more oscillators transmitting electromagnetic energy, providing one or more resonant circuits receiving electromagnetic energy from the oscillators, bringing the fluid and the one or more resonant circuits into contact with each other so that the receptions of the electro-magnetic energy of the resonant circuits are changed, and detecting changes of the transmissions of electromagnetic energy of the oscillators by changes in one or more characteristics thereof upon the changes in the receptions of the electromagnetic energy of the resonant circuits. The resonant circuits may consist of coils having separated windings made of an electrically conducting material, which may be provided by printing on a substrate. The resonant circuit(s) may be embedded in a diaper. Thus, WO 99/33037 discloses an absorbent article comprising at least one wetness detecting means.

Although electrical detecting means for indicating the status of an absorbent article have clear advantages over visual detecting means, they still suffer from the drawbacks of being expensive, stiff, bulky and difficult to incorporate into the article during manufacture. In addition, many traditional (silicon-based) electrical components are not readily disposable or degradable (e.g. batteries and PCBs). Traditional components of electrical circuits such as soldered metal are not compatible with materials such as paper, plastics and fibrous materials used in modern absorbent articles. Neither are they compatible with the rapid assembly-line manufacturing methods used in the production of absorbent articles. As absorbent articles are primarily intended for single use (i.e. they are disposable), it would be a great advantage if electrical detecting means were cheap and readily disposable. There is thus a need for a detecting means which can be readily incorporated into an absorbent article which provides the advantages of electrical detection, yet which is cost-effective, simple to manufacture and readily disposable.

SUMMARY OF THE INVENTION

The present invention addresses aforementioned problems associated with prior art absorbent articles in this technical field. The present invention concerns an absorbent article comprising at least one wetness detecting means.

The at least one wetness detecting means comprises at least one electrical circuit, said at least one electrical circuit being integrally formed into said article. The at least one electrical circuit is fabricated from an electrically active material which has been printed onto one or more components of the absorbent article.

In one embodiment of the present invention, the wetness detecting means is passive, i.e. it does not comprise a power source.

The at least one electrical circuit may comprise a capacitor and an inductor connected in parallel or series, wherein a change in the moisture-content of the absorbent article influences the resonant frequency of the electrical circuit. Suitably, a change in the moisture-content of the absorbent article influences the capacitance of the capacitor. The capacitor may comprise superabsorbent polymer between the plates of the capacitor. Furthermore, the capacitor may comprise a water-soluble substance between the plates of the capacitor.

In one aspect, a change in the moisture content of the absorbent article destroys the resonant frequency of the electrical circuit.

The electrical circuit may further comprise a sensor connected in parallel or in series with the capacitor and the inductor, wherein a change in the moisture-content of the absorbent article influences the conductance of a current across the sensor.

In another embodiment of the present invention, the wetness detecting means is active, i.e. it comprises a power source. According to this embodiment, the wetness detecting means may comprise at least one printed component selected from the group comprising a printed battery, a printed antenna, a printed memory circuit, a printed logic circuit and a printed sensor. The at least one printed component may be selected from the group comprising a printed battery, a printed antenna and a printed sensor.

The wetness detecting means according to the present invention may comprise a plurality of sensors located in different regions of the absorbent article. Furthermore the absorbent article may comprise a plurality of wetness detecting means located in different regions of the absorbent article.

## BRIEF DESCRIPTION OF THE DRAWINGS

Figure 1 shows an open diaper according to the Invention comprising an electrical detecting means.
Figures 1a - c show a first embodiment of the printed electrical circuit of the invention.
Figures 2a - b show a second embodiment of the printed electrical circuit of the invention.
Figures 3a - b show a third embodiment of the printed electrical circuit of the invention.
Figures 4 and 5 show a fourth embodiment of the printed electrical circuit of the invention.
Figure 6 shows a representation of the operation of the electrical detecting means of the invention.

## DETAILED DESCRIPTION OF PREFERRED EMBODIMENTS

*Absorbent Article*

The present invention concerns an absorbent article indicated generally by reference number 10 in Figure 1, such as a diaper, pant diaper, belted diaper or incontinence guard. The absorbent article comprises a liquid-permeable topsheet 12, a liquid-impermeable backsheet 14 and an absorbent core 16 located therebetween.

The liquid-permeable topsheet 12 optionally consists of a nonwoven material, e.g., a spunbond material of continuous filaments, a meltblown material, a bonded carded fibrous web or a perforated plastic film. Different types of laminates, e.g. laminates of non-woven material and plastic film may optionally also be used. Materials which are suitable for the liquid-permeable topsheet 12 should be soft and non-irritating to the skin. Furthermore, the topsheet 12 can be different in different portions of the article 10.

The liquid-impermeable backsheet 14 may consist of a plastic film, a nonwoven material treated with a liquid impervious material or a hydrophobic nonwoven material which resists liquid penetration. Other types of liquid-barrier-materials may of course also be used as the liquid-impermeable backsheet 14, such as e.g. closed-cell plastic foams, various liquid-barrier laminates etc. It is preferable that the liquid-impermeable backsheet 14 is permeable to air and vapour.

The topsheet 12 and the backsheet 14 have a somewhat greater extension in the plane than the absorbent core 16 and extend outside the edges thereof. The topsheet 12 and the backsheet 14 are connected to each other within the projecting portions thereof, e.g., by gluing or welding by heat or ultrasound.

The absorbent core 16 can be of any conventional kind. Examples of commonly-occurring absorbent materials are cellulosic fluff pulp, tissue layers, highly absorbent polymers (so-called *"superabsorbents"*), absorbent foam materials, absorbent nonwovens and the like. It is common to combine cellulosic fluff pulp with superabsorbents in an absorbent

body. It is also common to have absorbent bodies comprising layers of different material with different properties with respect to liquid acquisition capacity, liquid distribution capacity and storage capacity. The thin absorbent bodies which are common in incontinence guards often comprise a compressed mixed or layered structure of cellulosic fluff pulp and superabsorbent.

Absorbent articles such as diapers usually require some sort of fastening means 20 which holds the article 10 closed. Suitable fastening means 20 may be mechanical fasteners such as hook-and-loop type fasteners, adhesives such as pressure-sensitive adhesives or a combination of mechanical and adhesive fasteners.

If the absorbent article 10 is a belt diaper, it will comprise belt portions, such that the belt portions comprise a sole component of the waist region of the diaper. The belt portions are attached or fastened to the front or the rear portion of the article, and fasten to each other around the waist of the wearer. The article is then passed between the legs of the wearer and fastened to the belt portions via the other of the front or rear portion. Fastening means 20 as described above are present on the belt portions and on the front/rear portion so that the article 10 can be firmly closed. Application of the article 10 implemented in this way allows a wearer to easily apply the belt diaper themselves, and even allows a diaper to be changed while the user is standing up.

Elastic elements 18 may be present in the absorbent article 10 of the present invention, for example at the leg or waist openings. The nature and location of such elastic elements 18 are known to the skilled person and need not be discussed further here.

The absorbent article 10 according to the present invention comprises at least one wetness detecting means 30. This detecting means 30 typically has a first electrical characteristic before the absorbent article 10 is soiled, and a second electrical characteristic after a soiling event has occurred. The open diaper 10 according to the present invention comprising wetness detecting means 30 is shown in Figure 1 in cut-away view.

*Printable electrically active materials*

The present invention requires the use of electrically active materials 50 which can be printed. The term *"electrically active"* is used in the present context to mean materials which can conduct electrical charge and thus be used to fabricate electrical circuits or components thereof. Although a border between conductive and semi-conductive active materials is not clearly defined, the present invention includes both aspects, to the extent that they fulfil the requirements of the electrical circuit in question. Electrically active materials 50 are not restricted to pure materials, but include mixtures of electrically active materials 50, in whatever form.

The electrically active materials 50 are printable. This means that the materials 50 are stable in liquid or solution form (i.e. can be solution-processed) and can be applied to a surface (in this case, a component of the absorbent article) in a desired pattern or form, which pattern or form is then retained after the material dries or cools. The electrically active materials 50 should also have mechanical properties which make them tolerant to flexing and tension which might be present in the absorbent article 10. They should also be stable to the environment in which they are to be used (i.e. stable to humidity, sunlight, oxygen etc.). Printing can be carried out by standard techniques known in the art, such as laser printing, inkjet printing, thermal printing, screen printing, offset printing, relief print and rotogravure.

Conductive polymers are one class of electrically active materials 50 which are printable. Such polymers generally have structures in which the electrons are heavily delocalised, e.g. through $\pi$-bonds (double or triple bonds), aromatic systems or electron lone pairs which are included in the polymer chain. The electrons are therefore free to move along the polymer structure. The extent of delocalisation determines the degree of conductivity - polymers which have poorly delocalised electrons will be less conducting than those having continuous delocalisation across the entire polymer structure. Examples of conducting polymers are polyphenylene-vinylene (PPV), polyaniline (PANI) and polypyrrole (PPy). The structures of these polymers are given below.

PPV

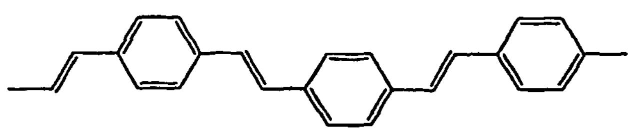

PANI

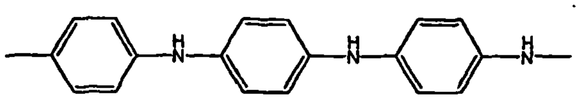

PPy

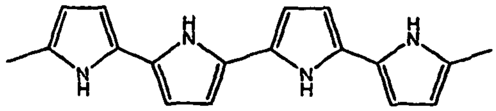

The electrical, mechanical and chemical properties of such polymers can be adjusted as desired, by, for example, cross-linking or substitution of the polymer, or combining them with other materials before printing. In many cases, these polymers need to be protected from air and humidity by laying a printed barrier film over the polymers, or by depositing the polymers simultaneously with a barrier matrix.

Another class of printable electrically active materials 50 are particle suspensions. These materials comprise small particles of an electrically-conducting material (e.g. a metal such as silver or copper, or a non-metal such as graphite) which are suspended in an organic solvent or carrier. The particles provide the material with the desired conductivity, while the organic solvent or carrier provides the required physical properties (e.g. plasticity, coefficient of thermal expansion, ease of application, viscosity and fracture toughness). The organic solvent or carrier may also contribute to the electrical properties of the electrically active material. The organic solvent may evaporate after printing, in which case, the particles remain on the printed surface. Alternatively, the organic carrier hardens after printing, so that the particles are trapped within the carrier. Examples of the latter embodiment are epoxy resins. Such particle suspensions are commercially available from DuPont electronics or TÄBY Sweden.

The printable electrically active materials 50 of the invention may comprise a mixture of the above-described particle suspensions and conductive polymers.

It is possible for different electrically active materials 50 to be printed in different regions or components of an absorbent article, depending on the type of electrical circuit which is required. By repeatedly printing electrically active materials 50 (optionally having different electrical properties) on the same region or component, it is possible to build up layers of electrically active material on top of each other. Alternatively, electrical components can be fabricated with intervening layers of the components of the absorbent article, so that a sandwich-type structure is created. This can be seen in the cut-away view in Fig. 1. The components of the absorbent article 10 may be selected or treated to be permeable or resistant to the electrically active material. All of these approaches allow complex electrical circuits to be manufactured.

*Printed electrical circuits*

The detecting means 30 according to the invention comprises an electrical circuit 40, which is integrally formed into said article 10, said at least one electrical circuit 40 being fabricated from an electrically active material 50 which has been printed onto one or more components of the absorbent article 10.

Electrical circuits fall into two general classes - active circuits, which comprise a power source as a component of the circuit, and passive circuits, which do not comprise a power source as one of their components, but act in response to an externally-applied power source.

By the phrase *"integrally formed",* it is meant that the electrical circuit 40 is an integral part of the absorbent article 10 and cannot be removed or disassembled without destroying either the absorbent article 10 or the electrical circuit 40 or both. In certain circumstances, the electrically active material 50 can penetrate into the components of the absorbent article 10.

Figure 1a shows a circuit diagram of a tuned circuit (also called an RLC circuit) which consists of a capacitor 60 and an inductor 70. There will naturally be a certain amount of resistance from the circuit; alternatively, resistors may be included in parallel or in series with the capacitor 60 and the inductor 70.

The capacitance C of a capacitor may be expressed mathematically as:

$$C = \frac{A\varepsilon_0\varepsilon_r}{d}$$

wherein A is the area of the capacitor plates (in $m^2$), *d* is the distance between the plates (in m), $\varepsilon_0$ is the permittivity of free space (ca. 8.8542 x $10^{-12}$ F/m) while $\varepsilon_r$ is the relative permittivity of a dielectric included between the capacitor plates, for example the capacitor 60 includes a layer 80 of dielectric between its plates 90. Cellulose in paper and cotton products has a relative permittivity of approximately 6.5. Unimpregnated dry tissue (kraft) paper has a relative permittivity of around 2.1. Polymers such as polyethylene and polypropylene have relative permittivities in a range of substantially 2.2 - 2.5. (Reference: Kaye & Laby, Tables of Physical and Chemical Constants, 15th ed. 1986)

At a simple level, the inductance *L* of an inductor is expressed mathematically as:

$$L = \frac{N^2 A \mu_0 \mu_r}{l}$$

wherein $\mu_0$ is the permeability of free space ($4\pi \times 10^{-7}$ Henries per metre), $\mu_r$ is the relative permeability of the core (dimensionless), $N$ is the number of turns in the inductor, $A$ is the cross sectional area of the inductor in square metres and I is the length in metres.

The resonance frequency $f_0$ of a tuned circuit can be calculated from the values of $L$ and $C$ via:

$$f_0 = \frac{1}{2\pi\sqrt{LC}}$$

Hence, upon application of an external RF field, a tuned circuit such as that illustrated in Fig. 1 a resonates at a natural resonant frequency, which can be adjusted through choice of capacitor and inductor variables listed above.

In one embodiment of the present invention, a change in the moisture-content of the absorbent article 10 influences the resonant frequency $f_0$ of the electrical circuit 40. There are a number of ways in which this might be achieved.

Firstly, the moisture content of the absorbent article 10 may influence the capacitance of the capacitor 60. For example, a water-swellable material may be present in the dielectric layer 80 between the plates 90 of the capacitor 60, so that, upon wetting, the distance d increases, and the capacitance decreases. Alternatively, liquid-absorbent material (such as superabsorbent polymer (SAP), cellulose or any other liquid-absorbent material) may be present in the dielectric layer 80 between the plates 90 of the capacitor 60. The absorption of liquid into the liquid-absorbent material has the effect of increasing the relative permittivity ($\varepsilon_r$) of the liquid-absorbent material (as water has a high relative permittivity), thus increasing the capacitance of the capacitor 60. As a further alternative, a water-soluble substance such as an inorganic salt may be present in the dielectric layer 80 between the plates 90 of the capacitor 60. The water-soluble substance dissolves upon contact with liquid and thus the capacitance of the capacitor 24 will change, and the resonant frequency $f_0$ of the circuit will alter. As a further alternative, a change in resistance of the circuit will change the resonant frequency $f_0$ of the circuit. A change in the moisture-content of the absorbent article 10 influences the resistance of the electrical circuit 40. This may be, for example, achieved by using conductive polymer materials protected by a water-soluble barrier film (e.g. an epoxy material). Upon wetting, the film dissolves and water, salts and urea will react with the conductive polymer material, changing the resistance of the circuit and thereby the resonant frequency $f_0$.

When printed onto a component of the absorbent article, such as a paper sheet or a plastic film, the circuit illustrated diagrammatically in Fig. 1a may be in the form shown in Fig. 1b. Figure 1b shows an inductor 70 which comprises a flat spiral printed in electrically active material 50 on a component of the absorbent article 10. The spiral typically has between 5-20 turns. The spiral has a first central area 110. A corresponding second central area 120 is printed on the opposite face of the component of the absorbent article - together these two central areas 110, 120 constitute the plates of the capacitor 90 which are separated by the component of the absorbent article 10. The circuit is completed by electrically active material which connects the second central area 120 to the outer end of the spiral, through the component of the absorbent article 10.

An alternative form for printing the circuit illustrated diagrammatically in Fig. 1a is shown in Fig. 1c. Figure 1c illustrates the inductor 70 which comprises a flat spiral form as in Figure 1b. The capacitor 60 is formed by first 160 and second 170 areas lying on opposite faces of the component of the absorbent article 10, outside the area comprised by the spiral. Together, these first 160 and second 170 areas constitute the plates of the capacitor 90.

An alternative way in which the moisture-content of the absorbent article 10 can influence the resonant frequency $f_0$ of the electrical circuit 40 is through destruction, namely disablement, of the electrical circuit 40. In other words, a change in the moisture content of the absorbent article 10 destroys the resonant frequency $f_0$ of the electrical circuit 40. This may be achieved through an electrical circuit as illustrated in Figure 2a. Such a circuit comprises a weak point 200, which for instance comprises water-soluble electrically active material, or electrically active material which is printed on a water-soluble component. An absorbent article comprising such the electrical circuit illustrated in Figure 2a will resonate at its resonant frequency upon application of an external RF field. Upon contact with a liquid, however, the electrical circuit 40 is physically broken and by virtue of the weak point 200 becoming a high-resistance or substantially open-circuit, the electrical circuit 40 will not then resonate upon application of an external RF field.

Figure 2b shows how the electrical circuit 40 illustrated diagrammatically in Fig. 2a may be printed. In its printed form, the electrical circuit 40 has essentially the same form as that shown in Figure 1b, with an inductor 70 which comprises a flat spiral and two central areas 300, 310 which constitute the plates 90 of the capacitor 60. The circuit shown in Figure 2b includes a weak point 200 which is broken upon contact with a liquid.

In a further embodiment, the electrical circuit 40 may comprise a sensor 400 connected in parallel or in series with the capacitor 60 and the inductor 70. A change in the moisture-content of the absorbent article 10 influences the conductance of a current across the sensor 400. A circuit diagram which illustrates the use of a sensor 400 is shown in Figure 3a.

Printed sensors 400 may take a number of forms. One possibility is to print the sensor 400 in a sandwich structure similar to those described for capacitors 24 above. This construction requires two layers of electrically active material 50 separated by a dielectric material. Alternatively, the sensor may have an interdigitated construction, in which electrically active material 50 is printed as a pair of "fork" shapes in which the prongs of the forks are interleaved, but without electrical contact between the prongs. This layout is advantageous, as it can be printed in one layer, making it less expensive to print than multiple layers. Hence, in one embodiment of the present invention, the wetness detecting means 30 is printed on a component of the absorbent article 10 which lies adjacent to the inner surface of a backsheet 14 of the absorbent article 10..

The principles involved in the sensor 400 are similar to those involved in the capacitor 60. Upon contact with liquid, the permittivity of the sensor 400 changes. This may be achieved by the physical dimensions or the relative permittivity of the sensor 400 changing upon contact with liquid. As a result, the resonant frequency $f_0$ of the tuned -circuit changes.

Figure 4 shows a circuit diagram of a more advanced electrical circuit, indicated generally by 500, which may make up the wetness detecting means 30. A major circuit comprises a first inductor 70a, a first capacitor 60a and a sensor 400 connected in parallel, with a diode 410 located in series with the first inductor 70a. The sensor 400 is connected in parallel via a transistor 420 with a minor circuit which is itself a tuned circuit comprising a second inductor 70b and a second capacitor 60b. The transistor 420 is further connected via a high resistance bias resistor 430and a third inductor 70c.

Operation of the more advanced electrical circuit 500 will now be described. In operation, the circuit 500 is subjected to an alternating magnetic field at a first frequency $f_1$: the first frequency is beneficially in a range of 10kHz to 100kHz. The first inductor 70a is arranged to include sufficient turns and be of sufficient area A so that a signal induced across the first inductor 70a has an amplitude in the order of a few volts. The diode 410 is operable to rectify the signal so as to generate a working supply potential in operation across the first capacitor 60a.

When the sensor 400 Is dry (i.e. a soiling event has not yet occurred), the transistor 420 of NPN type or MOS type is biased into a non-conducting state by virtue of the bias resistor 430; in such a non-conducting state, the transistor 420 is hindered from oscillating.

When a soiling event occurs, the sensor 400 becomes conductive, causing the transistor 420 to be biased into an active part of its characteristic: in consequence, positive feedback occurs between the second and third inductors 70b, 70c causing the transistor to oscillate at a frequency $f_2$ defined by the second inductor 70b and the second capacitor 60b. Optionally, the second frequency $f_2$ is substantially at least an order of magnitude greater than the frequency $f_1$ of exciting magnetic field applied

Figure 5 illustrates how the electrical circuit 500 of Figure 4 might be printed on an absorbent article 10. The first inductor 70a comprises a relatively large number of coils (e.g. 50 - 500), and may be printed together with the first capacitor 60a in the same way as the circuits illustrated in Figures 1-3. The sensor 400 may be printed in the same way as illustrated in Fig. 3. Diode 410 can be printed laying down multiple layers of electrically active material with selected electrical properties so as to build the required p-n junctions. Similarly, layers of electrically active material with different electrical properties can be used to build up transistor 420, either in MOS or bipolar implementation. Recently all-printed transistor devices with mobilities as high as 0.1-0.2 $cm^2/V$-s and on-off ratios as high as $10^4$ were reported (University of California, Berkeley). First, a gate electrode is printed onto a substrate using gold nanocrystals. This is followed by low-temperature annealing, and then polymer dielectric is deposited via inkjet printing. Source/drain contacts are then printed, again using gold nanocrystals.

The second and third inductors 70c, 70c comprise fewer coils than the first inductor (e.g. 5-20 coils) and may be printed together with the second capacitor 60b in the same way as the circuits illustrated in Figures 1-3. It is desirable that the first inductor 70a is distant from the second and third inductors 70b, 70c, so that coupling between the first inductor 70a and the second and third inductors 70b, 70c is minimised. For instance, the first inductor 70a could be located on the rear of the absorbent article 10, while the second and third inductors 70b, 70c are located on the front of the absorbent article 10. Suitably, the sensor 400 is printed in the crotch region of the absorbent article 10, which is the area in which wetting is easiest to detect. High frequency radiation at the frequency $f_2$ can be detected in an external detector device (e.g. transponder unit 700) which is selectively responsive to emitted radiation from the article 10 at the frequency $f_2$.

As an alternative to the above-described passive wetness detecting means 30, the wetness detecting means 30 may be active, i.e. it comprises a power source. Although such active wetness detecting means 30 are more complicated, they can provide a much wider functionality than passive wetness detecting means 18. A printed electrical circuit 40 can be divided into five major parts. These are printed batteries, printed antennae, printed memory circuits,

printed logic circuits and printed sensors. The most essential components are printed batteries, printed antennae and printed sensors.

Printed batteries comprise an electrolyte sandwiched between two electrodes. In printed batteries, the electrolyte is usually in the form of a gel which is sealed so as to avoid leakage. Suitable electrolytes are carbon-zinc electrolytes or zinc-manganese dioxide. One possible structure for a printed battery is alternating layers of zinc and manganese dioxide-based cathode and anode layers. Printed batteries may have a thickness which is generally between 0.5 and 1mm, and, if circular in form, a diameter which is between 25 and 50mm. Typical output voltages are 1.5V; the same as many conventional batteries. They are manufactured by standardised printing, drying and laminating equipment and processes. Printed batteries are commercially available from e.g. PowerPaper Ltd. of Israel or Thin Battery Technology (TBT) Inc of the USA. The battery itself may function as a sensor. Printed batteries can be made in such a way that they are inactive until contacted by a liquid. Upon activation (wetting), the battery sends a current to a circuit including one or more antennae. This generates an RF signal. Such batteries remove the need for a separate sensor, and are storage-stable.

Antennae are available as printed antennae, inlays or completed labels. Antennae are commonly printed with silver-based particle suspensions, such as those described above, which are compatible with both paper and polymer components of an absorbent article. Such antennae can provide performance to match traditional copper or aluminium antennae. An example of a commercially available printed antenna is FleX Wing produced by Precisia LLP.

Active wetness detecting means 30 may be designed to ensure long life of the battery, e.g. by pulsing the power supplied by the battery, or by using the battery only to power the memory and using a passive wetness detecting means 30 for generating an RF signal. The printed logic circuit may be used to monitor the printed sensor at given time intervals and save the result in the printed memory. Additionally, if the active wetness detecting means 30 comprises more than one printed sensor in different locations in the absorbent article 10, the logic circuit can be used to compare the signals from the sensors and gather data on the nature, extent and location of the wetness in the absorbent article 10. Particularly of interest are wetness detecting means 30 which provide a quantitative measure of the status of an absorbent article, rather than a simple on/off measure. Printed memory circuits can be used to maintain a record of the status of the absorbent article 10. Preferably, the memory does not require constant power supply.

The absorbent articles 10 of the present invention are used in combination with an RF transmitter/receiver (transponder) unit 700 (Fig. 6). The transponder unit 700 comprises an inductor coil which generates an RF field; an antenna which detects an RF signal generated by an electrical circuit 40; indicating means such as one or more audible sounders, indicator lights, display screen etc.; a power source (e.g. batteries) and circuitry for controlling the inductor coil, the antenna and the indicating means. The indicating means may be a loudspeaker which generates an audible signal or an LED which lights up when the absorbent article 10 needs to be changed. Alternatively, the status of an absorbent article 10 may be displayed on a display screen which forms part of the transponder unit 700. The transponder unit 700 is preferably a portable hand-held device, so that the status of a wearer's absorbent article 10 can be determined easily and quickly without disturbing the wearer.

The transponder unit 700 generates an RF field which corresponds to the resonant frequency of the electrical circuit 40. The electrical circuit 40 resonates, and the RF signal thus produced can be detected by the transponder unit 700. The RF signal generated by the electrical circuit 40 is optionally beneficially in the region 10 - 100kHz. In order to be able to detect even weak RF signals generated by the electrical circuit 40, it is advantageous that the RF field generated by the transponder unit is pulsed, so that any weak RF signals generated by the electrical circuit 40 are not obscured by the RF field generated by the transponder unit 700. The electrical circuit 40 will continue to resonate for a short while after the RF field generated by the transponder unit 700 is interrupted, so that weak RF signals can be detected at the unit 700. It may be advantageous for the transponder unit 700 to include a threshold, below which an RF signal generated by the electrical circuit 40 will not activate the indicating means. This will provide advantages in that an absorbent article 10 need not be changed after every soiling event, but rather the caregiver can wait until a certain level of wetness has been reached.

The transponder unit 700 may be arranged so as to scan a range of frequencies. In this way, small deviations in the resonant frequency of the electrical circuit 40 can be accommodated. Additionally, by scanning a range of frequencies, the progression of an electrical circuit 40 from an initial resonant frequency to a final resonant frequency can be seen, which also allows the caregiver to wait until a certain level of wetness has been reached before changing the absorbent article.

Optionally, the transponder unit 700 comprises a memory unit, in which data concerning the number of times an absorbent article is changed can be stored. This information can be downloaded to a computer and then used by caregivers to determine statistics or to make predictions for future consumption of absorbent articles. Furthermore, if a particular electrical circuit 40 provides a particular resonant frequency which can be correlated with a particular wearer, wearer-specific data can be gathered.

The wetness detecting means 30 of the invention may comprise a plurality of sensors 400 located in mutually different regions of the absorbent article 10. In this way, the nature, extent and location of the wetness in the absorbent

article 10 can be monitored. It is preferred that the sensors 400 are located in the crotch region of the absorbent article 10, where wetness is most likely to be detected. Additionally or alternatively, the absorbent article 10 may comprise a plurality of wetness detecting means 30 located in mutually different regions of the absorbent article 10. If the wetness detecting means 30 is of the "short-circuit" type (as shown and described in Fig. 2A-2B), it is preferably located in the crotch region of the absorbent article, where wetness is most likely to be detected. If, however, the wetness detecting means 30 is not of this type (e.g. if it is moisture-sensitive) it is advantageous for it not to be located in the crotch portion, so that it is not saturated immediately upon soiling of the absorbent article 10.

The present invention should not be limited by the above description and embodiments, but rather by the appended claims. In particular, features which are shown in connection with one embodiment may be combined with or replaced by features from one or more other embodiments.

## Claims

1. An absorbent article (10) comprising at least one wetness detecting means (30), wherein
said at least one wetness detecting means (30) comprises at least one electrical circuit (40), said at least one electrical circuit (40) being integrally formed into said article (10), said at least one electrical circuit (40) being fabricated from an electrically active material (50) which has been printed onto one or more components of the absorbent article (10), **characterised in that** the wetness detecting means (30) is active, i.e. it comprises a power source.

2. An absorbent article according to claim 1, **characterised In that** the electrical circuit (40) comprises a capacitor (60) and an inductor (70) connected in parallel or series, wherein a change In the moisture-content of the absorbent article (10) influences the resonant frequency of the electrical circuit (40).

3. An absorbent article according to claim 2, **characterised In that** a change In the moisture-content of the absorbent article (10) Influences the capacitance of the capacitor (60).

4. An absorbent article according to claim 3, **characterised In that** the capacitor (60) comprises liquid-absorbent material (80) included between the plates (90) of the capacitor (60).

5. An absorbent article according to claim 3, **characterised In that** the capacitor (60) comprises a water-soluble substance (80) included between the plates (90) of the capacitor (60).

6. An absorbent article according to any one of claims 1-5, **characterised In that** a change In the moisture-content of the absorbent article (10) influences the resistance of the electrical circuit (40).

7. An absorbent article according to claim 2, **characterised In that** a change In the moisture content of the absorbent article (10) destroys the resonant frequency of the electrical circuit (40).

8. An absorbent article according to any one of claims 2-7, **characterised in that** the electrical circuit (40) further comprises a sensor (400) connected In parallel or In series with the capacitor (60) and the inductor (70), wherein a change In the moisture-content of the absorbent article (10) influences the conductance of a current across the sensor (400).

9. An absorbent article according to claim 1, **characterised in that** the wetness detecting means (30) comprises at least one printed component selected from the group comprising a printed battery, a printed antenna, a printed memory circuit, a printed logic circuit and a printed sensor.

10. An absorbent article according to claim 9, **characterised In that** the at least one printed component Is selected from the group comprising: a printed battery, a printed antenna and a printed sensor.

11. An absorbent article according to any one of claims 1-10, **characterised in that** the wetness detecting means (30) comprises a plurality of sensors (400) located In mutually different regions of the absorbent article (10).

12. An absorbent article according to any one of claims 1-11, **characterised In that** the absorbent article (10) comprises a plurality of wetness detecting means (30) located In mutually different regions of the absorbent article (10).

13. An absorbent article according to any one of claims 1-12, the wetness detecting means (30) is printed on a component of the absorbent article (10) which lies adjacent to the Inner surface of a backsheet (14) of the absorbent article (10).

14. An absorbent article according to any one of claims 1-6 or 8-12, wherein the wetness detecting means (30) provides a quantitative measure of the status of an absorbent article (10).

**Patentansprüche**

1. Absorbierender Artikel (10), der mindestens ein Mittel zur Feuchtedetektion (30) umfasst, wobei das mindestens eine Mittel zur Feuchtedetektion (30) mindestens einen elektrischen Schaltkreis (40) umfasst, wobei der mindestens eine elektrische Schaltkreis (40) in den Artikel (10) integriert ist und der mindestens eine elektrische Schaltkreis (40) aus einem elektrisch aktiven Material (50) hergestellt ist, das auf eine oder mehrere Komponenten des absorbierenden Artikels (10) gedruckt worden ist,
**dadurch gekennzeichnet, dass**
das Mittel zur Feuchtedetektion (30) aktiv ist, d.h., eine Stromquelle umfasst.

2. Absorbierender Artikel nach Anspruch 1, **dadurch gekennzeichnet, dass** der elektrische Schaltkreis (40) einen Kondensator (60) und ein Induktionselement (70) umfasst, die parallel oder in Reihe geschaltet sind, wobei eine Änderung des Feuchtegehalts des absorbierenden Artikels (10) die Resonanzfrequenz des elektrischen Schaltkreises (40) beeinflusst.

3. Absorbierender Artikel nach Anspruch 2, **dadurch gekennzeichnet, dass** eine Änderung des Feuchtegehalts des absorbierenden Artikels (10) die Kapazität des Kondensators (60) beeinflusst.

4. Absorbierender Artikel nach Anspruch 3, **dadurch gekennzeichnet, dass** der Kondensator (60) ein Flüssigkeitabsorbierendes Material (80) umfasst, das zwischen den Platten (90) des Kondensators (60) eingeschlossen ist.

5. Absorbierender Artikel nach Anspruch 3, **dadurch gekennzeichnet, dass** der Kondensator (60) eine wasserlösliche Substanz (80) umfasst, die zwischen den Platten (90) des Kondensators (60) eingeschlossen ist.

6. Absorbierender Artikel nach mindestens einem der Ansprüche 1 bis 5, **dadurch gekennzeichnet, dass** eine Änderung des Feuchtegehalts des absorbierenden Artikels (10) den Widerstand des elektrischen Schaltkreises (40) beeinflusst.

7. Absorbierender Artikel nach Anspruch 2, **dadurch gekennzeichnet, dass** eine Änderung des Feuchtegehalts des absorbierenden Artikels (10) die Resonanzfrequenz des elektrischen Schaltkreises (40) zerstört.

8. Absorbierender Artikel nach mindestens einem der Ansprüche 2 bis 7, **dadurch gekennzeichnet, dass** der elektrische Schaltkreis (40) ferner einen Sensor (400) umfasst, der parallel oder in Reihe mit dem Kondensator (60) und dem Induktionselement (70) geschaltet ist, wobei eine Änderung des Feuchtegehalts des absorbierenden Artikels (10) die Leitfähigkeit eines Stroms durch den Sensor (400) beeinflusst.

9. Absorbierender Artikel nach Anspruch 1, **dadurch gekennzeichnet, dass** das Mittel zur Feuchtedetektion (30) mindestens eine gedruckte Komponente umfasst, die aus der Gruppe ausgewählt ist, die eine gedruckte Batterie, eine gedruckte Antenne, einen gedruckten Speicherschaltkreis, einen gedruckten Logikschaltkreis und einen gedruckten Sensor umfasst.

10. Absorbierender Artikel nach Anspruch 9, **dadurch gekennzeichnet, dass** die mindestens eine gedruckte Komponente aus der Gruppe ausgewählt ist, die eine gedruckte Batterie, eine gedruckte Antenne und einen gedruckten Sensor umfasst.

11. Absorbierender Artikel nach mindestens einem der Ansprüche 1 bis 10, **dadurch gekennzeichnet, dass** das Mittel zur Feuchtedetektion (30) mehrere Sensoren (400) umfasst, die in verschiedenen Regionen des absorbierenden Artikels (10) angeordnet sind.

12. Absorbierender Artikel nach mindestens einem der Ansprüche 1 bis 11, **dadurch gekennzeichnet, dass** der absorbierende Artikel (10) mehrere Mittel zur Feuchtedetektion (30) umfasst, die in verschiedenen Regionen des

absorbierenden Artikels (10) angeordnet sind.

13. Absorbierender Artikel nach mindestens einem der Ansprüche 1 bis 12, **dadurch gekennzeichnet, dass** das Mittel zur Feuchtedetektion (30) auf eine Komponente des absorbierenden Artikels (10) gedruckt ist, die zu der inneren Oberfläche eines Backsheets (14) des absorbierenden Artikels (10) benachbart ist.

14. Absorbierender Artikel nach mindestens einem der Ansprüche 1 bis 6 oder 8 bis 12, in dem das Mittel zur Feuchtedetektion (30) eine quantitative Messung des Zustandes des absorbierenden Artikels (10) gewährleistet.

## Revendications

1. Article absorbant (10) comportant au moins un moyen de détection d'humidité (30), dans lequel ledit moyen de détection d'humidité (30) comprend au moins un circuit électrique (40,), ledit circuit électrique (40) au minimum unique étant réalisé d'un seul tenant dans ledit article (10), ledit circuit électrique (40) au minimum unique étant fabriqué à partir d'un matériau actif électriquement (50) qui a été imprimé sur un ou plusieurs composants de l'article absorbant (10), **caractérisé en ce que** le moyen de détection d'humidité (30) est actif, par exemple du fait qu'il comprend une source d'énergie.

2. Article absorbant selon la revendication 1 **caractérisé en ce que** le circuit électrique (40) comprend un condensateur (60) et un inducteur (70) reliés en parallèle ou en série, une variation de la teneur en humidité de l'article absorbant (10) ayant une influence sur la fréquence de résonance du circuit électrique (40).

3. Article absorbant selon la revendication 2 **caractérisé en ce qu'**une variation de la teneur en humidité de l'article absorbant (10) a une influence sur la capacité du condensateur (60).

4. Article absorbant selon la revendication 3 **caractérisé en ce que** le condensateur (60) comprend un matériau (80) absorbant les liquides inclus entre les plaques (90) du condensateur (60).

5. Article absorbant selon la revendication 3 **caractérisé en ce que** le condensateur (60) comprend une substance (80) soluble dans l'eau incluse entre les plaques (90) du condensateur (60).

6. Article absorbant selon l'une quelconque des revendications 1 à 5 **caractérisé en ce qu'**une variation de la teneur en humidité de l'article absorbant (10) a une influence sur la résistance du circuit électrique (40).

7. Article absorbant selon la revendication 2 **caractérisé en ce qu'**une variation de la teneur en humidité de l'article absorbant (10) détruit la fréquence de résonance du circuit électrique (40).

8. Article absorbant selon l'une quelconque des revendications 2 à 7 **caractérisé en ce que** le circuit électrique (40) comprend en outre un capteur (400) connecté en parallèle ou en série au condensateur (60) et à l'inducteur (70), une variation de la teneur en humidité de l'article absorbant (10) ayant une influence sur la conduction d'un courant traversant le capteur (400).

9. Article absorbant selon la revendication 1 **caractérisé en ce que** le moyen de détection d'humidité (30) comporte au moins un composant imprimé choisi dans le groupe comprenant : un accumulateur imprimé, une antenne imprimée, un circuit de mémoire imprimé, un circuit logique imprimé et un capteur imprimé.

10. Article absorbant selon la revendication 9 **caractérisé en ce que** le composant imprimé au minimum unique est choisi dans le groupe comprenant : un accumulateur imprimé, une antenne imprimée et un capteur imprimé.

11. Article absorbant selon l'une quelconque des revendications 1 à 10 **caractérisé en ce que** le moyen de détection d'humidité (30) comprend une pluralité de capteurs (400) placés dans des zones différentes les unes des autres de l'article absorbant (10).

12. Article absorbant selon l'une des revendications 1 à 11 **caractérisé en ce que** l'article absorbant (10) comprend une pluralité de moyens de détection d'humidité (30) placés dans des zones différentes les unes des autres de l'article absorbant (10).

13. Article absorbant selon l'une quelconque des revendications 1 à 12 **caractérisé en ce que** le moyen de détection d'humidité (30) est imprimé sur un composant de l'article absorbant (10) qui est situé à proximité de la surface intérieure d'une feuille arrière (14) de l'article absorbant (10).

14. Article absorbant selon l'une quelconque des revendications 1 à 6 ou 8 à 12 dans lequel le moyen de détection d'humidité (30) donne une mesure quantitative de l'état d'un article absorbant (10).

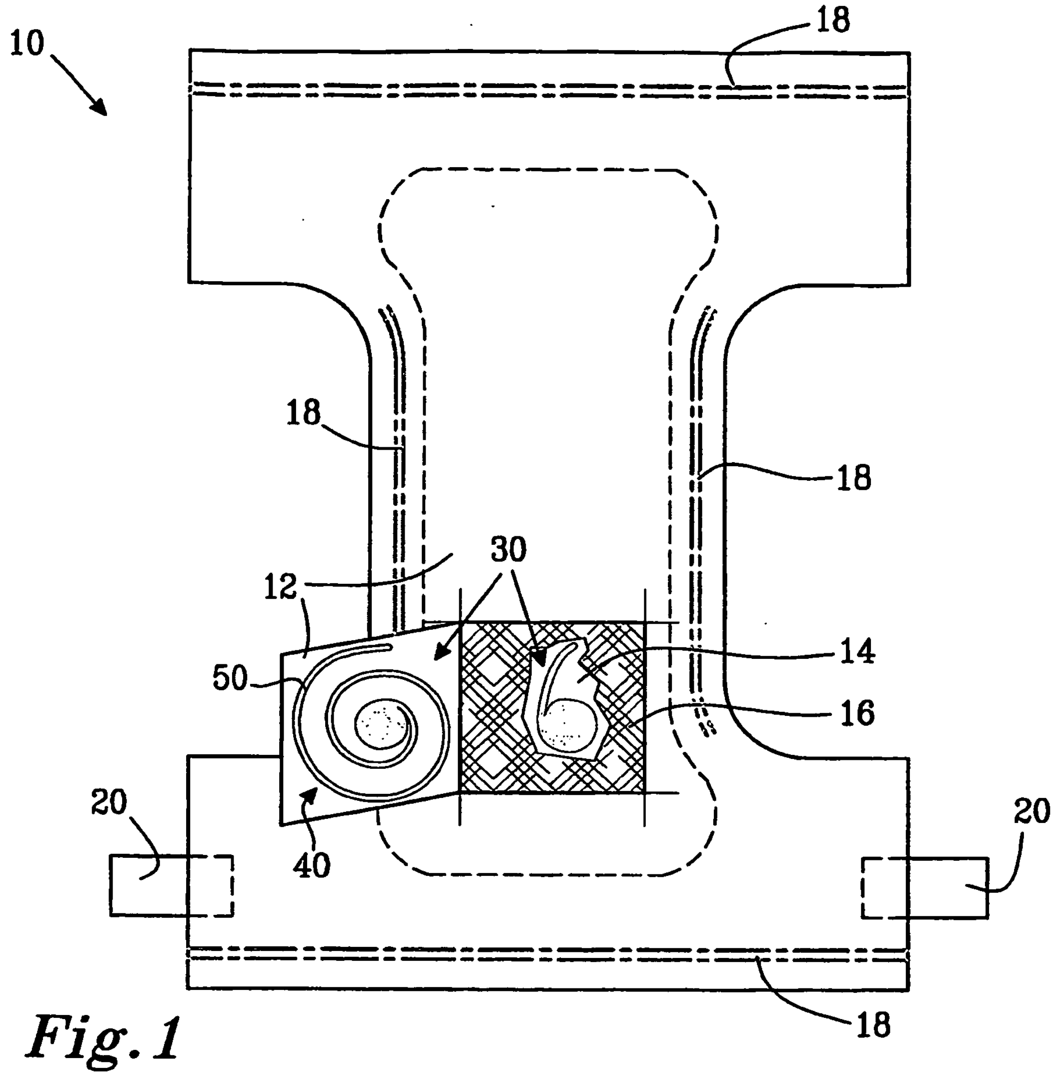


Fig. 1

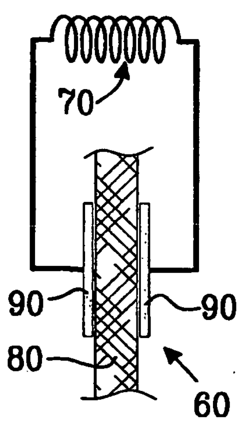


Fig. 1A

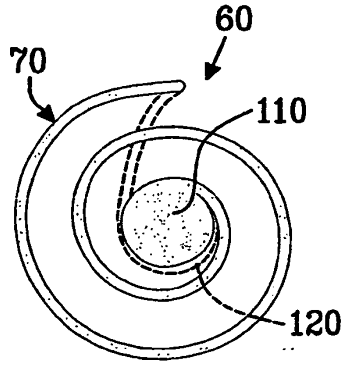


Fig. 1B

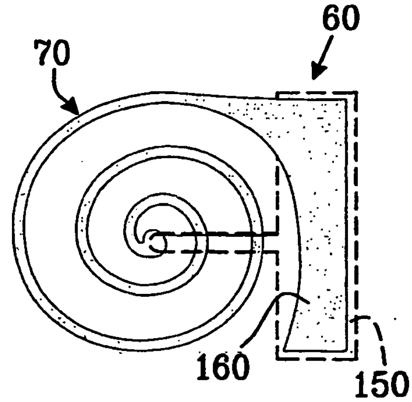


Fig. 1C

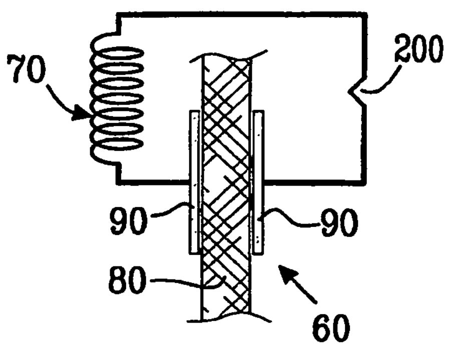

Fig.2A

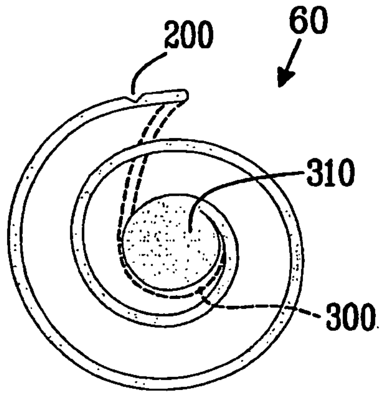

Fig.2B

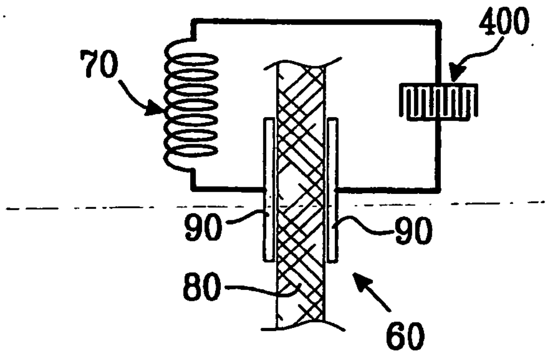

Fig.3A

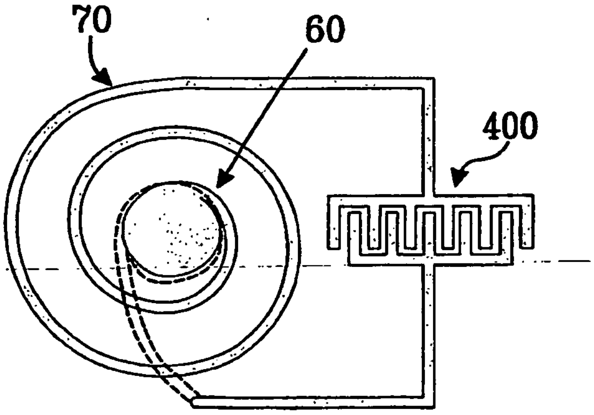

Fig.3B

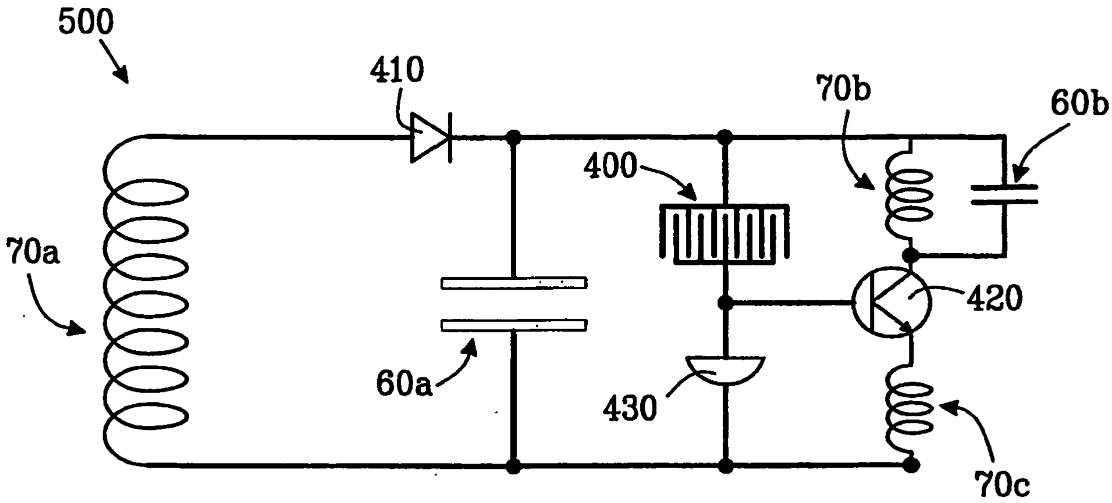
500
410
70b
60b
400
70a
420
60a
430
70c

*Fig.4*

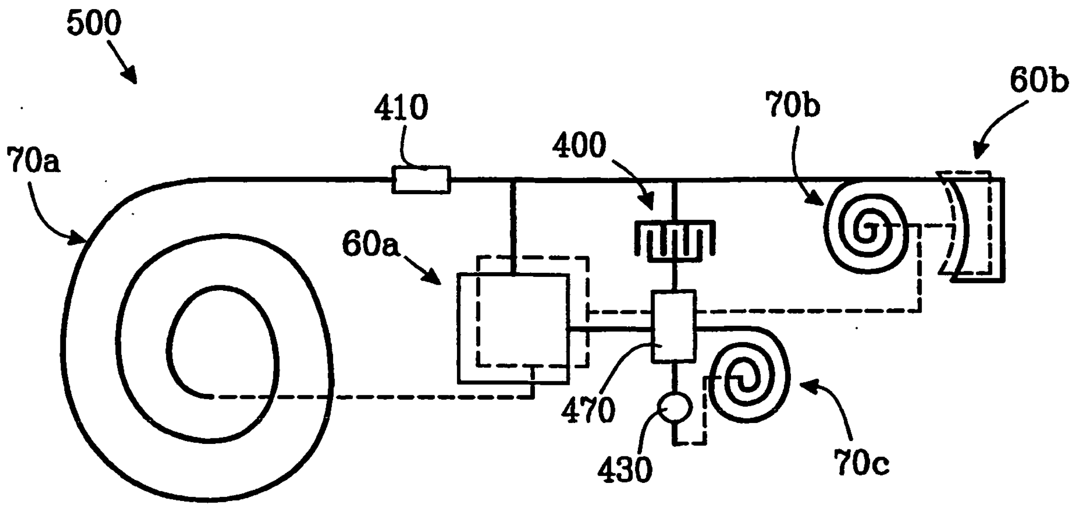
500
410
70b
60b
70a
400
60a
470
430
70c

*Fig.5*

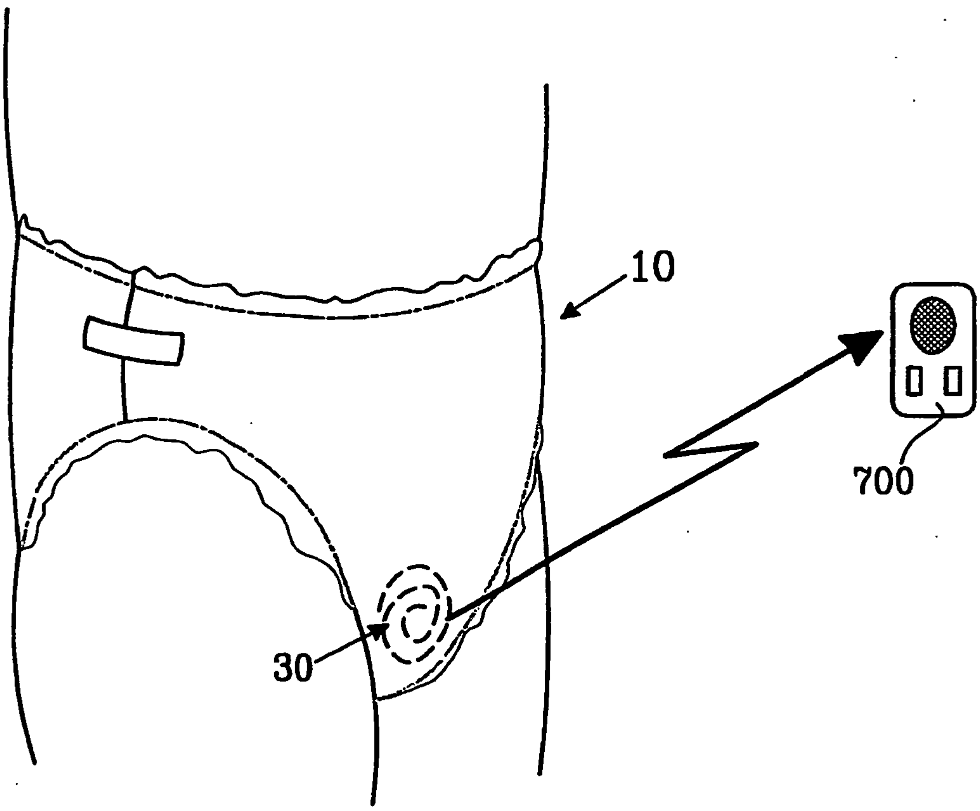


Fig.6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5389093 A
- WO 04028403 A
- WO 05030084 A
- WO 0247592 A
- WO 0278513 A
- JP 2005000602 B
- WO 9933037 A


### Non-patent literature cited in the description


- **KAYE ; LABY.** Tables of Physical and Chemical Constants. 1986